# EUROPEAN PATENT SPECIFICATION

(11) **EP 3 406 296 B1**
(45) Date of publication and mention of the grant of the patent: **22.03.2023**
(21) Application number: 18173281.9
(22) Date of filing: 18.05.2018
(51) Int. Cl.: A61N 1/36, A61N 1/05, H04R 25/00

(54) **FITTING DEVICE AND SYSTEM FOR FITTING A COCHLEAR IMPLANT**
PASSVORRICHTUNG UND SYSTEM ZUR PASSUNG EINES COCHLEAR-IMPLANTATS
DISPOSITIF D'AJUSTEMENT ET SYSTÈME D'AJUSTEMENT D'UN IMPLANT COCHLÉAIRE

(30) Priority: 24.05.2017 EP 17172727
(43) Date of publication of application: 28.11.2018
(73) Proprietor: Oticon Medical A/S, 2765 Smørum (DK)
(72) Inventor: PEDERSEN, Regin Kopp, 2765 Smørum (DK)
(74) Representative: Demant

(56) References cited:
- WO-A1-96/39005
- WO-A1-2007/112737
- WO-A2-2012/101495
- US-A1- 2009 043 359
- US-B1- 8 923 979

## Description

### FIELD

The disclosure refers to cochlear implants. In particular, the disclosure relates to a fitting device and a system for fitting a cochlear implant.

### BACKGROUND

Cochlear implants are hearing assistance devices that are surgically implanted electronic device that provides a sense of sound to a person who is profoundly deaf or severely hard of hearing.

Hearing aids capture sound, convert it into electric sound signals, amplify and filter the electric signals, convert the electric signals in amplified sound and send the amplified through the normal auditory channel. These hearing aids are designed for people with slight to moderately severe perceptive hearing loss.

In cases of severe to profound hearing loss, these hearing aids are not powerful enough. If the ear damage is too severe, amplifying sound using a traditional hearing aid are generally not helpful. The cochlear implant overcomes this by sending a signal derived from captured sound directly to the auditory nerve. Unlike traditional hearing aids, cochlear implants bypass the damaged areas of the ear. They capture the sound, process electric sound signals and electrically stimulate the auditory nerve.

Currently, fitting of cochlear implants is currently done using fitting software that directly adjust the stimulation levels of the physical electrodes of the electrode array that has been inserted into the cochlear. Such adjustment is usually made on either electrode by electrode basis or on selected electrodes with intermediate electrodes getting thresholds determined by linear interpolation. It is up to the person doing the fitting (the fitter) to translate the acquired knowledge about the hearing into individual electrode adjustments that compensates hearing challenges at specific audio frequencies. To do this, the professional needs to manually correlate the frequency range associated to each electrode to the frequency that is desired to be adjusted and then make adjustments that secures smooth threshold profiles across the electrodes/frequency spectrum. In another words, the fitter needs to manually relate frequency related audiological test results such as narrow band/warble tone audiometry and speech testing to the physical electrodes based on the frequency windows associated to each of the physical electrodes. The frequency windows are only available as numeric attributes to the electrodes.

The classical way of setting map level is the following procedure, which is based on single-electrode psychophysics:
A person that carries out the fitting (a fitter) stimulates a specific electrode with a burst of pulses that occurs at the desired rate. This stimulation starts at a very low level and the stimulation is slowly increased in small steps. The recipient is asked to report any auditory sensation. The stimulation is increased until the recipient hears the sound, then, using finer steps, an up-down procedure (very similar to audiometry) is used to find the threshold of sensation for this burst. The level at the threshold is used as T-level. Once the T-level is established, the fitter increases the stimulation in small steps until the recipient indicates that the sound is loud but comfortable. This is used as C-level.

The above procedure is repeated for all electrodes on the array.

When all electrodes are tested, generally a balancing procedure is performed to make sure that all C-levels generate approximately the same loudness, which, in turn, ensures that equal-levelled audio input generates equal-levelled electrical stimulation.

When this is done, the implant is put into speech processing mode, and the map is tested with speech input. If needed the fitter can lower or raise the T- and/or C-levels to adjust the loudness perception of live speech.

A system and a method for fitting a cochlear implant is disclosed in WO 2012/101495 and WO 96/39005.

Furthermore, free field audiometry is often used to evaluate hearing with a cochlear implant. Audiometry provides threshold and loudness scaling results related to standard audiometric frequency bands (250, 500, 1k, 2k, 4k, ...) and inter octave bands. These results can guide the fitter to adjust the stimulation settings relating to those bands accordingly. The fitter needs to manually identify which cochlear implant electrodes to adjust (and how much) based on the electrode frequency distribution (which is available as numbers only). Some electrodes may be contained within an audiometric frequency band while others may lie on the boundary or not be affected at all. This is a time consuming exercise.

In addition, comparison of loudness sensation is often performed by stimulating single electrodes in sequence asking the patient if they sound alike in intensity. There is currently no means of comparing loudness sensation between various broader frequency bands like e.g. audiometric frequencies involving multiple electrode stimulations.

The disclosure aims at providing a fitter to easily and efficiently program cochlear implants.

### SUMMARY

Typical cochlear implant systems have of two parts:
- an internal part (A) that is a receiver surgically implanted in the temporal bone underneath the skin, and an electrode array placed in the cochlea, and
- an external part (B) that is a behind-the-ear part which comprises a sound processor and a lead connecting the sound processor to an antenna. The antenna is magnetically attached to the skin over the internal part and allows for wireless data communication between the external part and the internal part.

The external part typically comprises
- one or more microphones that pick up sound from the environment,
- a sound processor which selectively filters sound to prioritize audible speech,
- a transmitter that sends power and the processed sound signals across the skin to the internal device by electromagnetic induction.

The internal part typically comprises:
- a receiver/stimulator, which receives signals from the sound processor and converts them into electric impulses,
- an electrode array embedded in the cochlea.

When in use, the sound processor processes electric sound signals representing sound picked up by the microphone(s). The transmitter is magnetically attached to the skin. It transmits the digitised sound from the sound processor to the implant receiver. The magnetic implant receiver/stimulator is fitted under the skin directly under the antenna. It transforms the digital information into electric stimulation signals to be applied by electrodes of an electrode array placed in the cochlea. The electrode array is inserted in the cochlea. Each electrode on the array corresponds to a signal frequency. The auditory nerve is stimulated when the electric stimulation signal is transmitted to the corresponding electrode. The stimulation of the auditory nerve is perceived as sound by the patient's brain.

The sound processor is configured using fitting software that creates custom programs for each user.

According to a first aspect, a fitting device for fitting a cochlea implant that has a plurality of stimulation electrodes configured to emit stimulation pulses is disclosed. The fitting device comprises a display unit, an interface unit for data communication with a cochlear implant and input means for entering parameter values for fitting the cochlea implant data to be displayed on the display device. The fitting device is configured
to communicate data to and/or from a cochlear implant, the data comprising, for each stimulation electrode of the cochlear implant, data defining a frequency range and data representing at least two stimulation intensity levels, including a threshold level (T-level) for each individual stimulation electrode of the cochlear implant; and
to display on the display, for each electrode, a stimulation intensity range and a frequency range in a two dimensional matrix wherein one dimension is assigned to frequency whereas the other dimension is assigned to stimulation intensity, and where said parameter values include a minimum intensity level and a maximum comfortable stimulation intensity level, where the minimum intensity level denotes the minimum intensity where the user will notice a sound sensation caused by the stimulation pulses, and the maximum intensity level denotes the maximum stimulation that can be applied without causing discomfort with the user, wherein the stimulation intensity ranges and the frequency ranges of all electrodes are displayed simultaneously.
to read out said input means and altering said parameter values in response to user inputs and updating said graphic representation accordingly, and the graphic representation is generated in a live mode, wherein the graphical representation indicates momentary the stimulation intensity range in real time and frequency contents of a sound being presented to the patient, and
to automatically adapt further parameter values in response to one of the user inputs that causes a manipulation of one parameter value, resulting in smooth parameter adjustments of neighbouring parameters.

According to a another aspect, a system for fitting a cochlea implant that has a plurality of stimulation electrodes configured to emit stimulation pulses is disclosed. The system comprises a fitting device and a cochlear implant communicatively connected to the fitting device. The cochlear implant comprises an electrode lead with a plurality of stimulation electrodes forming an electrode array, a stimulation unit to generate stimulation pulses to be emitted via individual stimulation electrodes in controlled manner with an adjustable stimulation intensity and a sound processing unit for generating a stimulation intensity control signal for each electrode depending on frequency ranges assigned to each individual electrode and a respective signal strength of an input signal in a respective frequency range.

According to an embodiment, the fitting device comprises a graphical display and a user interface providing input means. The fitting device is configured to
communicate data to and/or from the cochlear implant, wherein the data represent, for each stimulation electrode of the cochlear implant, parameter values defining a frequency range and parameter values defining at least two stimulation intensity levels, including a maximum comfortable stimulation intensity level and a minimum threshold level (T-level) for each individual stimulation electrode of the cochlear implant, where the minimum intensity level denotes the minimum intensity where the user will notice a sound sensation caused by the stimulation pulses, and the maximum intensity level denotes the maximum stimulation that can be applied without causing discomfort with the user,
display on the display, for each electrode, a graphic representation of the parameter values that define a stimulation intensity range and a frequency range in a two dimensional matrix wherein one dimension is assigned to frequency whereas the other dimension is assigned to stimulation intensity, wherein the stimulation intensity ranges and the frequency ranges of all electrodes are displayed simultaneously,
read out said input means and altering said parameter values in response to user inputs and update said graphic representation accordingly, and the graphic representation is generated in a live mode, wherein the graphical representation indicates momentary the stimulation intensity range in real time and frequency contents of a sound being presented to the patient, and to
automatically adapt further parameter values in response to one of the user inputs that causes a manipulation of one parameter value, resulting in smooth parameter adjustments of neighbouring parameters.

Adjusting electrodes levels are often associated with some uncertainty as hearing thresholds and comfortable levels are determined from subjective testing requiring consistent responses from the patient to difficult sound level judgements. The fitter therefore often choose to estimate the levels (C and T) of a limited set of inserted electrodes and estimates the values of the others by interpolation or experience. A smooth electrode level profile is typically assumed. Adjustment/fine tuning of selected electrode levels during the course of the fitting therefore typically involve manual adjustment of neighbouring electrode levels as well to maintain smooth T and C level profiles across the electrode array. Such adjustments are currently supported by tools such as linear interpolation between selected electrodes or a selection of predefined profile shaping options. These methods are quite locked in the way the apply profile changes and do not offer freedom to adjust in a smooth way exactly where the fitter finds it needed. Automatic adjustment of neighbouring parameters can therefore facilitate the fitting procedure.

Said graphic representation may be a graphical user interface.

Said fitting device may be configured to generate a graphical user interface comprising a graphic representation of said parameter values that define a stimulation intensity range and a frequency range for an individual stimulation electrode, wherein the parameters assigned to an individual electrode are represented by a rectangle wherein one dimension of said rectangle represents the frequency range assigned to said electrode on a logarithmic scale whereas the other dimension represents the stimulation intensity range on a linear scale.

Said fitting device may further be configured to display in real time a volume unit meter representing the input sound level measured by the cochlear implant sound processor during fitting. Presently, during fitting, the fitter tests if the patient can hear various sounds and how loud they are perceived. Current cochlear implant fitting software have no sound level meter showing the fitter how loud sounds are at the ear level of the patient and it is therefore fitter subjective how loud the sound should actually sound to the patient. This disadvantage can be overcome by displaying a volume unit meter representing the input sound level measured by the cochlear implant sound processor during fitting.

Said fitting device may further be configured to provide and display a time line indicator with an index movable along a bar, said index being movable by a user wherein moving said index causes displaying representations of previous parameter values. Such time line indicator is beneficial because the fitting of a cochlear implant often change over time due to adaptation and changing physical and physiological properties. Monitoring of the physical and physiological changes is provided via objective measures assessing:
I. the electrical characteristics of the coupling between electrode array and cochlear tissue (impedance testing)
II. the generated nerve response as function of the applied stimulus levels (Electrical Compound Action Potential - ECAP)
III. the contraction of the stapedius muscle in the middle ear as function of the applied stimulus levels (Stapedius reflex test - SRT)

The objective tests can help reveal system failures (disconnected electrodes, short circuited electrodes), electrode array dislocations (movement in or extraction of the electrode from the cochlear over time). It is therefore important for the fitter to monitor changes in objective measures over time. In current software, there is no easy and fast way to scroll through and compare historic test results with the latest one. This is overcome by providing a time line indicator with an index movable along a bar, wherein the index is movable by a user wherein moving the index causes displaying representations of previous parameter values.

Said fitting device may be configured to detect user input that corresponds to dragging graphical components of said graphic representation and to alter parameter values in response to a detected dragging event and to update said graphic representation accordingly.

Said fitting device may further be configured to display in real time a power spectrum of an acoustic input signal within the graphic representation of the parameter values.

The system and the fitting device according to an embodiment allow to generate a graphical representation of the configuration of the cochlear implant system wherein the major parameters to be set for each electrode during fitting, namely the frequency range and the stimulation intensity range for the electrode, are represented in a single graphical representation. This graphical representation provides a direct visual relation between input sound frequencies and electrodes.

### BRIEF DESCRIPTION OF THE ACCOMPANYING FIGURES

The following description of embodiments of the disclosure illustrates various features and functions of the cochlea implant fitting system with reference to the accompanying figures.
- Fig. 1: illustrates a cochlea implant system comprising an external part and an internal part according to an embodiment of the disclosure;
- Fig. 2: is a schematic illustration of components of the cochlea implant system parts according to an embodiment of the disclosure;
- Fig. 3: illustrates the parts involved in fitting a cochlea implant including a fitting device according to an embodiment of the disclosure;
- Fig. 4: illustrates an exemplary display content of the fitting device according to an embodiment of the disclosure;
- Fig. 5: illustrates another display content of the fitting device during fitting in a live mode according to an embodiment of the disclosure;
- Fig. 6: illustrates still another display content of the fitting device during fitting according to an embodiment of the disclosure;
- Fig. 7: illustrates an additional volume unit meter (VU meter) according to an embodiment of the disclosure;
- Fig. 8a: illustrates a timeline function according to an embodiment of the disclosure;
- Fig. 8b: illustrates a timeline function according to an embodiment of the disclosure;
- Fig. 9: illustrates smooth fitting of a cochlea implant according to an embodiment of the disclosure; and
- Fig. 10: is a flow chart illustrating a method for fitting cochlear implant which is not a part of the disclosure.

### DETAILED DESCRIPTION

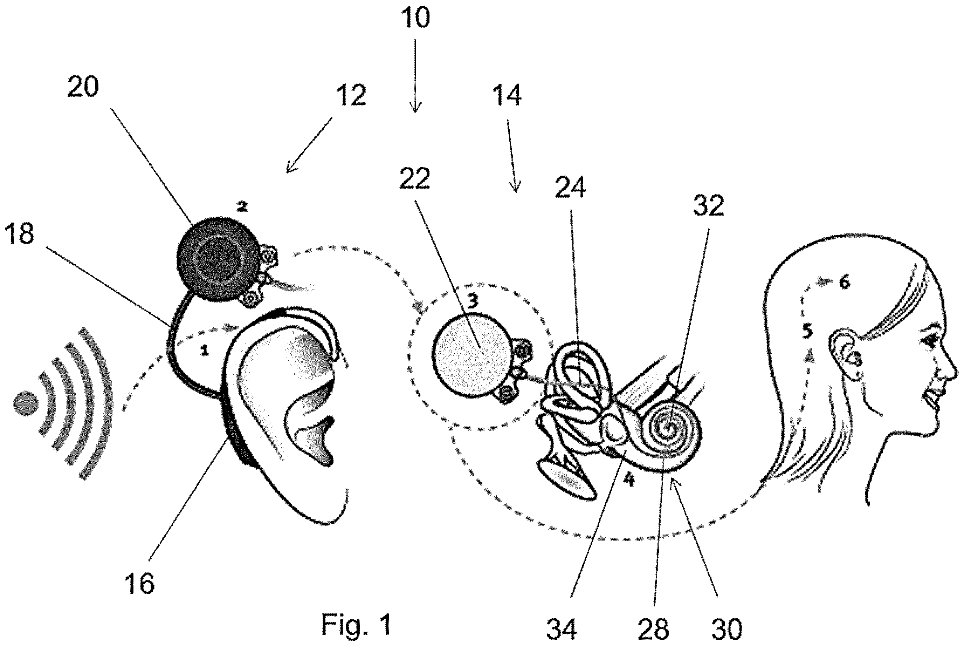

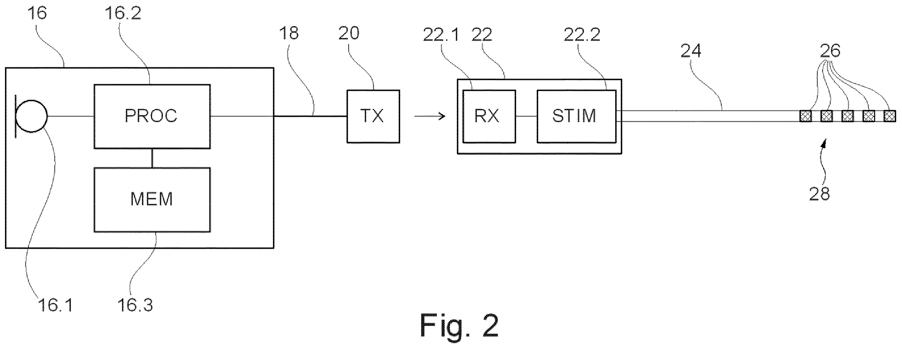

As disclosed in Figs. 1 and 2, a typical cochlea implant system 10 comprises an external part 12 and an internal part 14.

The external part 12 includes a behind-the-ear part 16 that comprises one or more microphones 16.1 that pick up sound from the environment. A sound picked up by the microphone 16.1 is converted into electrical sound signals that can be processed by a sound processor 16.2 that in turn generates an electric output signal. The sound processor 16.2 is arranged within the behind-the-ear part 16 and is connected to or comprises a memory 16.3 for storing inter alia parameter values. The electric output signal is fed via a lead 18 to a transmitter 20.

The internal part 14 of the cochlea implant system is implanted. It comprises a receiver/stimulator 22 that in use is arranged opposite to transmitter 20. The receiver/stimulator 22 comprises a receiver unit 22.1 that is connected to a stimulation unit 22.2 that generates stimulation signals in accordance with signals received from transmitter 20. The stimulation unit 22.2 is electrically connected to an electrode lead 24 that has a number of about 20 electrodes 26 arranged along a distal part 28 of electrode lead 24. The distal part 28 of electrode lead 24 extends along the cochlea 30.

It is noted that, in a healthy ear, nerves close to an apex 32 of the cochlea 30 will sense low frequency sound whereas nerves closer to the base 34 of cochlea 30 will sense higher frequency sound. Accordingly, stimulation electrodes closer to the distal end of electrode lead 24 and thus closer to the apex 32 of cochlea 30 are meant to stimulate nerves that, in a healthy ear, would sense higher frequency sound whereas stimulation electrodes closer to receiver/stimulator 22 and thus also closer to the base 34 of cochlea 30 are arranged to stimulate nerves that would otherwise sense lower frequency sound.

Each electrode 26 on electrode lead 24 can emit stimulation pulses (or a train of pulses) with adjustable stimulation intensity. It is noted that a user will notice a sound sensation caused by stimulation pulses emitted by a particular electrode only if the stimulation intensity of that electrode exceeds a minimum stimulation intensity level. This stimulation intensity level is called "threshold level" or "T-level". Likewise, for each electrode there is a maximum stimulation intensity that can be applied without causing discomfort with the patient. This maximum comfortable stimulation intensity level is called "comfort level" or "C-level".

The sound processor in the behind-the-ear part 16 of the cochlea implant system is configured to generate stimulation intensity control signals for each electrode on electrode lead 24.

During fitting of the cochlear implant, the sound processor is programmed to convert incoming electric sound signals into stimulation intensity control signals to transmitted to the receiver/stimulator 22 of the internal part 14. During fitting, inter alia the threshold-level and the comfort-level can be adjusted for each electrode and each electrode pulse can be assigned to a particular frequency range. The stimulation intensity control signal for a particular electrode depends on a momentary sound level of incoming sound in the frequency range assigned to that particular stimulation electrode. If the sound level in a certain frequency range of incoming acoustic sound is low, than the stimulation intensity applied by the electrode assigned to that frequency range should be low. The sound processor is configured to generate the stimulation intensity control signal accordingly.

The detailed configuration of the sound processor is achieved during fitting.

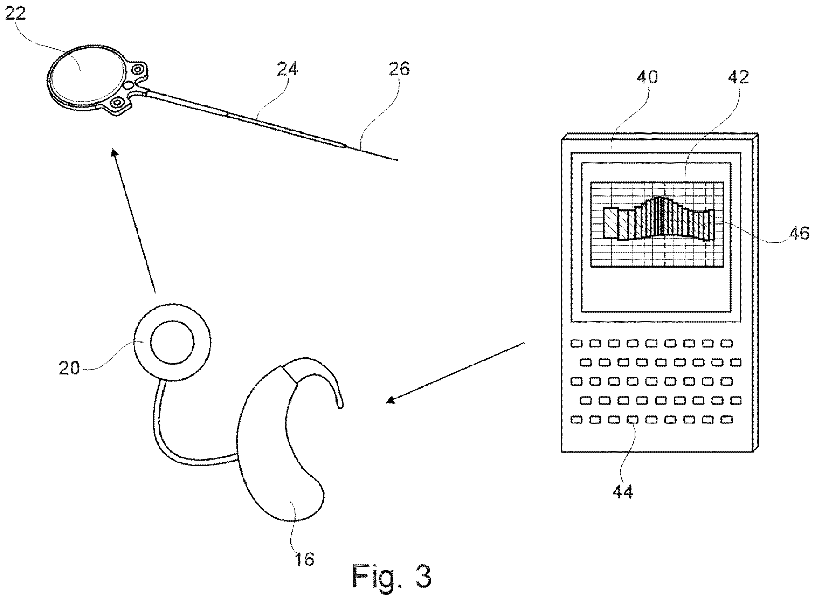

Fig. 3 illustrates a fitting system including a fitting device 40 according to an embodiment of the disclosure.

For fitting the cochlear implant 10, the fitting device 40 has a graphics display 42 and a user interface 44 that allows a fitter to interact with the fitting device 40.

The graphics display 42 may be touch sensitive to thus provide another user interface.

In an embodiment, the fitting device 40 includes a computer having a display and input means that may include conventionally known input means like keyboard or a pointer device like mouse.

The fitting device 40 is configured to communicate with the sound processor in the behind-the-ear part 16 of cochlear implant 10. The sound processor in the behind-the-ear part 16 of cochlear implant 10 in turn may communicate with the receiver/stimulator 22 via lead 18 and transmitter 20. The receiver/stimulator 22 causes delivery of stimulation pulses via one or more of the electrodes on the distal part 26 of electrode lead 24 in according with stimulation intensity control signals generated by the sound processor in the behind-the-ear part 16.

The fitting device 40 is further configured to generate a graphic representation of a configuration of a cochlear implant system 10 to be fitted.

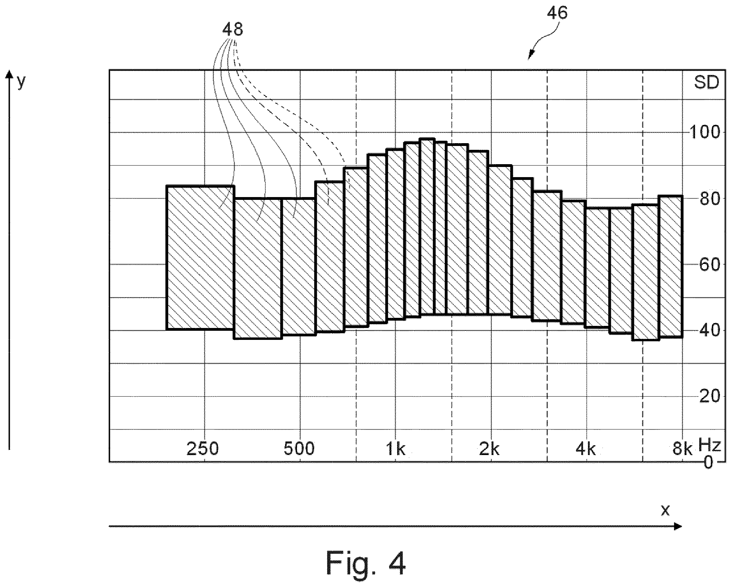

According to an embodiment, the fitting device 40 is configured to display on display 42, for each electrode, a stimulation intensity range and a frequency range in a two dimensional matrix wherein one dimension is assigned to frequency whereas the other dimension is assigned to stimulation intensity, wherein the stimulation intensity ranges and the frequency ranges of all electrodes are displayed simultaneously; see Figure 4.

The fitting device 40 is configured to depict a bar for each active electrode in a graphical representation with a logarithmic horizontal frequency axis as with audiograms. Accordingly, each electrode is represented by a rectangular bar 48. The top of the bar represents the C value that corresponds to the comfort level. The bottom of each bar represents the T value that corresponds to the comfort level.

The left side of each bar represents the lower frequency associated to the particular electrode and the right side of the bar the higher frequency associated to the particular electrode.

Accordingly, fitting device 40 is configured to generate a graphical representation 46 of the configuration of the cochlear implant system 10 wherein the major parameters to be set for each electrode during fitting, namely the frequency range and the stimulation intensity range for the electrode, is represented in a single graphical representation. This graphical user interface is new and unique to cochlear implant fitting software and provides a direct visual relation between input sound frequencies, electrodes, along with T and C levels within a particular frequency range.

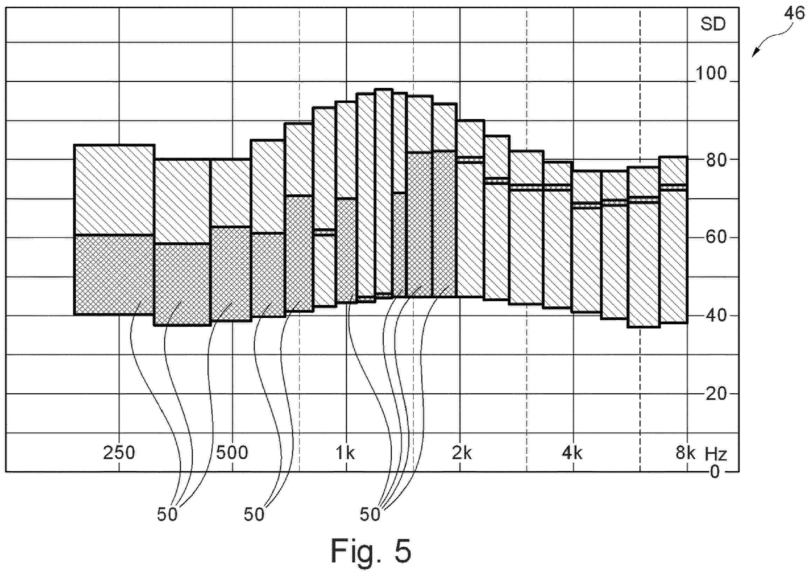

Fitting device 40 is further configured to generate a graphical representation in a live mode wherein the graphical representation can directly indicate the momentary stimulation levels in close to real time and due to the use of the frequency axis also visualise the frequency contents of the sound being presented to the patient; see Figure 5. Accordingly, a power spectrum 50 of incoming acoustic sound is displayed within the graphical representation 46 of the parameter values defining the stimulation intensity ranges and frequency ranges of the electrodes. Such display is applicable only for frequency ranges that are comprised in the incoming acoustic sound.

In a further preferred embodiment (not shown) the frequency spectrum of the sound is displayed in different, e.g. higher frequency resolution than the electrodes represent. For instance, the output of the Fast Fourier Transformation (FFT) before grouping into electrodes may be displayed. This could potentially help the fitter to see if the electrode frequency allocation could be adjusted to allow the patient to better discriminate specific sounds with different frequency spectra.

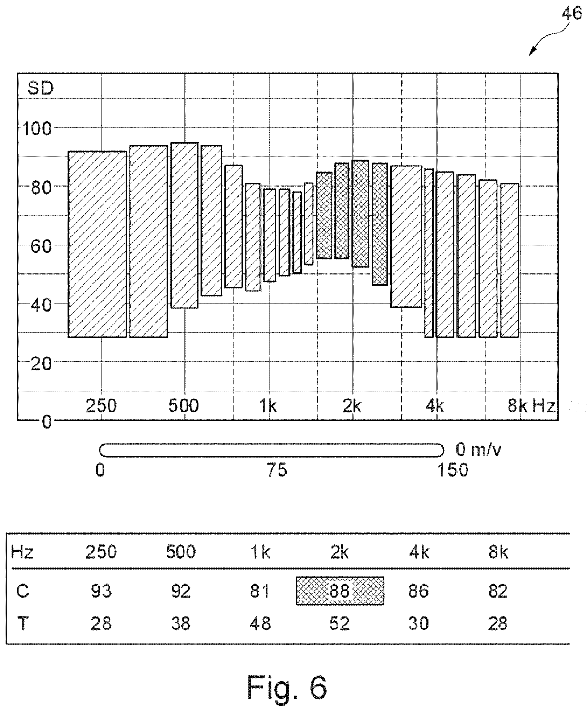

The fitting device 40 is further configured to support fitting in that the fitting device 40 supports adjustments of electrode settings relating to predefined audiometric frequencies directly; see Figure 6.

The fitting device 40 provides the fitter direct access to T and C level adjustments (and possibly other electrode properties) that are not electrode but frequency specific. When selecting and adjusting levels this way, the fitting system automatically maps the frequency interval to the electrodes based on the defined frequency distribution. The mapping allows automatic selection of electrodes for stimulation when using calibrated stimulation (single tone and sweep) and for adjustments. An implementation of automatic mapping may be a table similar to those used in hearing aid fitting software but with T and C levels instead of gain settings. This allows the fitter to easily select and adjust stimulation levels relating to the audiometric frequencies as found relevant based on e.g. aided free field audiometry testing and loudness scaling results.

When selecting multiple frequency bands for sweep stimulation, the corresponding sets of automatically selected electrodes can be stimulated in sequence providing an easy and fast loudness perception assessment across the audiometric frequency bands.

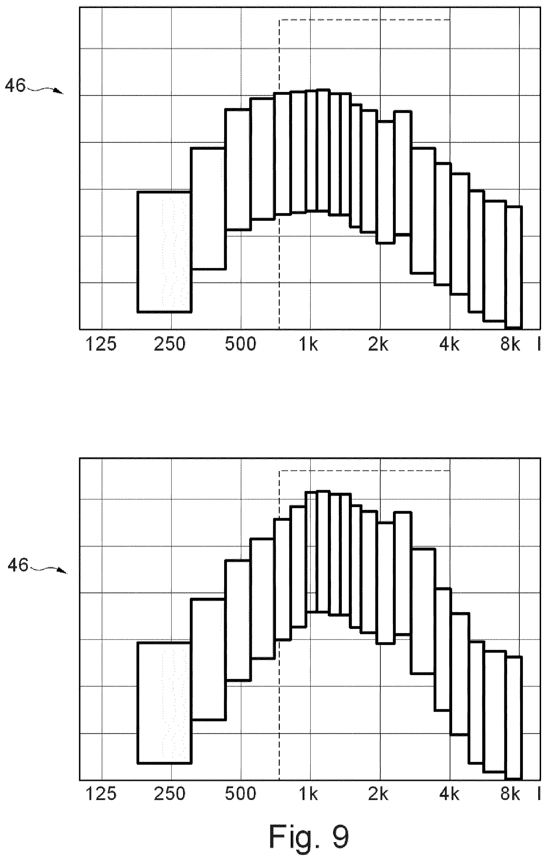

Audiometric adjustments will have to take into account that the electrode frequency allocation may not match the audiometric frequency bands. This may be handled using the smooth adjustment principle described with respect to figure 9.

In a further preferred embodiment, the fitting device may be configured to allow selection and adjustment of frequency intervals that are not directly represented in the audiometric table. To allow such selection, the fitting device may be further configured to allow the fitter to drag over a frequency range in the frequency related graph. Adjustments will then be based on that frequency range rather than the predefined ranges represented in the audiometric table.

In a further preferred embodiment, the fitting device is configured to provide a recommended or automatic adjustment of T and C values based on available audiometric test results.

Further, the fitting device may be configured to allow adjustment of two cochlear implants of a binaural cochlear implant system in that the two electrode arrays in a bilateral set up can be adjusted in an interlinked manner or separately.

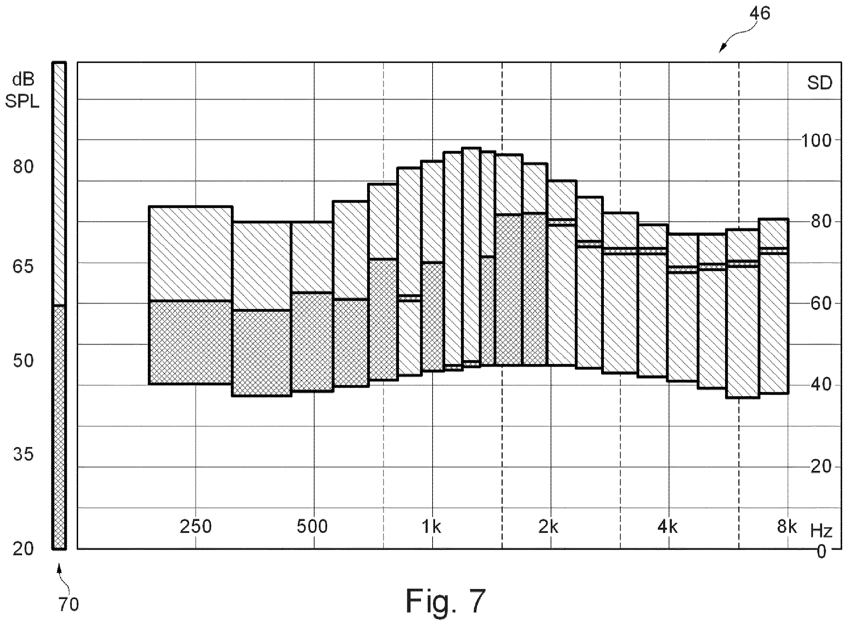

Still further, the fitting device may be configured to represent a volume unit meter 70 (VU-meter) as shown in Figure 7. The VU meter is illustrated in Figure 7 by the vertical dB SPL bar, which indicates the input sound level at a microphone of sound processor the patient. This may be useful in particular in a remote fitting situation where the input sound level is transmitted to the fitting software.

The VU meter 70 showing the input sound level (broadband or narrow band) is introduced to show the sound level measured by the cochlear implant sound processor during live mode allowing the fitter to monitor his/her own voice level at the ear level of the patient as well as the level of any other sound around the patient, even in cases where the patient may be sitting in another location as it is the case in remote fittings. This allows for a direct comparison of input sound levels and resulting stimulation levels that can help the fitter to see the effect of various sound processing feature settings, input AGCs, output compression systems, N of M, noise reduction etc.

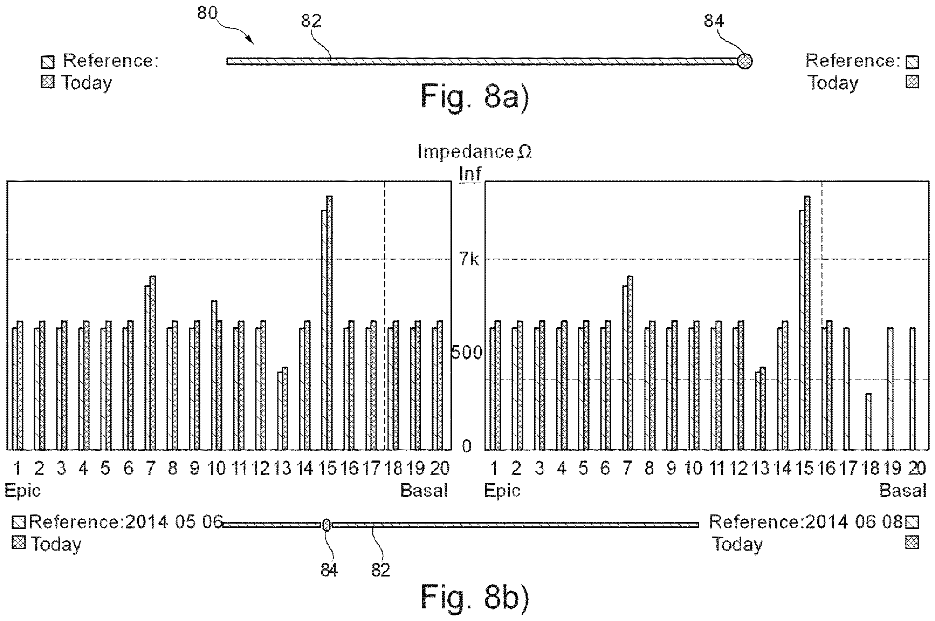

Figures 8a and 8b illustrate a timeline slider 80 provided by a preferred embodiment of fitting device 40. The timeline slider 80 can be used to quickly and dynamically scroll through a history of objective measurement results. Comprises a bar 82 and a sliding index 84 that can be moved by a user.

Points on the timeline represent previous objective measurements (may be linked to patient visits/sessions) that the fitter wants to compare the latest measurement results with. When the timeline slider is over a historic measurement, the results of that historic measurement can then be overlaid with the results of the latest measurement in a graph (e.g. by a grey side bar to impedance test results as in figure 8) to visualise the changes. Sliding it fast back and forth will produce a movie like visualisation of the changes in measurement results without the need to look into tables or numerous graphs. An example is shown in Figure 8b.

In yet another preferred embodiment, the fitting device 40 is configured to provide a smooth adjustment of the T-level, the C-level or both; see Figure 9.

The implementation of a smooth adjustment for T, C or T+C electrode settings allows the fitter to adjust any selected frequency range (or group of electrodes) while automatically maintaining a smooth electrode level profile. This is achieved by applying the adjustment at full strength at the centre of the selected frequency interval! electrodes but gradually at less strength near and around the lower and upper frequency borderlines of the selection. This way, adjustments are done in a smooth manner that gently pushes the level of the existing electrode level profile in a rubber band manner without smoothing out details of the profile. This means that any inter electrode level fluctuations/differences are automatically preserved to the extent possible. This saves the fitter for time consuming manual adjustments of neighbour electrodes.

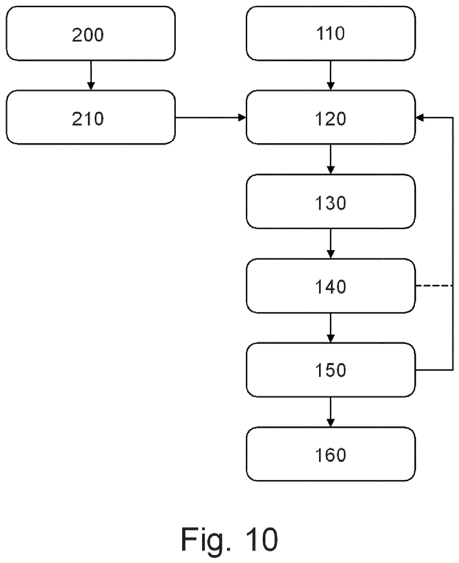

Figure 10 is a flow chart illustrating a preferred method 100 of fitting a cochlear implant. The method comprises the steps of:
communicating data to and/or from a cochlear implant, wherein the data represent, for each stimulation electrode of the cochlear implant, parameter values defining a frequency range and parameter values defining at least two stimulation intensity levels, including a threshold level (T-level) for each individual stimulation electrode of the cochlear implant (step 110) and
displaying on the display, for each electrode, a graphic representation of the parameter values that define a stimulation intensity range and a frequency range in a two dimensional matrix wherein one dimension is assigned to frequency whereas the other dimension is assigned to stimulation intensity, wherein the stimulation intensity ranges and the frequency ranges of all electrodes are displayed simultaneously (step 120).

The method further comprises monitoring the user interface and detecting user input that relates to altering parameter values (step 130). In response to detecting a user input event parameter values are altered (step 140) and the graphic representation is updated accordingly (step 120).

Optionally, step of altering parameter values directly as given by the detected user input, further parameter values may be alters automatically in order to achieve a smooth adaptation of neighbouring parameters (step (150).

Optionally, the method may comprise receiving an electric sound signal (step 200) and generate a graphical representation (step 210) in a live mode wherein the graphical representation represents a power spectrum of the incoming electric sound signal. The graphical representation of the power spectrum is integrated into the graphical representation of the parameter values that define a stimulation intensity range and a frequency range (step 120).

The method further may include programming adjusted parameter values into a memory 16.3 of the cochlear implant system (step 160).

Further optional method steps are apparent from the detailed description above.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope is defined by the claims that follow.

## Claims

1. A system for fitting a cochlea implant that has a plurality of stimulation electrodes configured to emit stimulation pulses, said system comprising a fitting device and a cochlear implant communicatively connected to said fitting device, wherein said cochlear implant comprises an electrode lead with a plurality of stimulation electrodes forming an electrode array, a stimulation unit to generate stimulation pulses to be emitted via individual stimulation electrodes in controlled manner with an adjustable stimulation intensity and a sound processing unit for generating a stimulation intensity control signal for each electrode depending on frequency ranges assigned to each individual electrode and a respective signal strength of an input signal in a respective frequency range, wherein
said fitting device comprises a graphical display and a user interface providing input means, said fitting device being configured to:
communicate data to and/or from said cochlear implant, said data representing, for each stimulation electrode of the cochlear implant, parameter values defining a frequency range and parameter values defining at least two stimulation intensity levels, including a maximum comfortable stimulation intensity level and a minimum threshold level (T-level) for each individual stimulation electrode of the cochlear implant, where the minimum intensity level denotes the minimum intensity where the user will notice a sound sensation caused by the stimulation pulses, and the maximum intensity level denotes the maximum stimulation that can be applied without causing discomfort with the user,
display on said display, for each electrode, a graphic representation of said parameter values that define a stimulation intensity range and a frequency range in a two dimensional matrix wherein one dimension is assigned to frequency whereas the other dimension is assigned to stimulation intensity, wherein the stimulation intensity ranges and the frequency ranges of all electrodes are displayed simultaneously,
read out said input means and altering said parameter values in response to user inputs and update said graphic representation accordingly, and the graphic representation is generated in a live mode, wherein the graphical representation indicates momentary the stimulation intensity range in real time and frequency contents of a sound being presented to the patient, and to
automatically adapt further parameter values in response to one of the user inputs that causes a manipulation of one parameter value, resulting in smooth parameter adjustments of neighbouring parameters.

2. A system according to claim 1, wherein said graphic representation is a graphical user interface.

3. A system according to claim 1 or 2, wherein said fitting device is configured to generate a graphical user interface comprising a graphic representation of said parameter values that define a stimulation intensity range and a frequency range for an individual stimulation electrode, wherein the parameters assigned to an individual electrode are represented by a rectangle wherein one dimension of said rectangle represents the frequency range assigned to said electrode on a logarithmic scale whereas the other dimension represents the stimulation intensity range on a linear scale.

4. A system according to at least one of claims 1 to 3, wherein said fitting device is further configured to display in real time a volume unit meter representing the input sound level measured by the cochlear implant sound processor during fitting.

5. A system according to at least one of claims 1 to 4, wherein said fitting device is further configured to provide and display a time line indicator with an index movable along a bar, said index being movable by a user wherein moving said index causes displaying representations of previous parameter values.

6. A system according to at least one of claim 1 to 5, wherein said fitting device is configured to detect user input that corresponds to dragging graphical components of said graphic representation and to alter parameter values in response to a detected dragging event and to update said graphic representation accordingly.

7. A fitting device for fitting a cochlea implant that has a plurality of stimulation electrodes configured to emit stimulation pulses, said fitting device comprising a display unit, an interface unit for data communication with a cochlear implant, input means for entering parameter values for fitting the cochlea implant data to be displayed on said display device, said fitting device being configured
to communicate data to and/or from a cochlear implant, said data comprising, for each stimulation electrode of the cochlear implant, data defining a frequency range and data representing at least two stimulation intensity levels, including a threshold level (T-level) for each individual stimulation electrode of the cochlear implant and
to display on said display, for each electrode, a stimulation intensity range and a frequency range in a two dimensional matrix wherein one dimension is assigned to frequency whereas the other dimension is assigned to stimulation intensity, and where said parameter values include a minimum intensity level and a maximum comfortable stimulation intensity level, where the minimum intensity level denotes the minimum intensity where the user will notice a sound sensation caused by the stimulation pulses, and the maximum intensity level denotes the maximum stimulation that can be applied without causing discomfort with the user, wherein the stimulation intensity ranges and the frequency ranges of all electrodes are displayed simultaneously,
to read out said input means and altering said parameter values in response to user inputs and updating said graphic representation accordingly, and the graphic representation is generated in a live mode, wherein the graphical representation indicates momentary the stimulation intensity range in real time and frequency contents of a sound being presented to the patient, and
to automatically adapt further parameter values in response to one of the user inputs that causes a manipulation of one parameter value, resulting in smooth parameter adjustments of neighbouring parameters.

## Patentansprüche

1. System zur Passung eines Cochlea-Implantats, das eine Vielzahl von Stimulationselektroden aufweist, die dazu konfiguriert sind, Stimulationsimpulse abzugeben, das System umfassend eine Passvorrichtung und ein Cochlea-Implantat, das mit der Passvorrichtung kommunikativ verbunden sind, wobei das Cochlea-Implantat eine Elektrodenleitung mit einer Vielzahl von Stimulationselektroden, die eine Elektrodenanordnung bilden, eine Stimulationseinheit zum Erzeugen von Stimulationsimpulsen, die über individuelle Stimulationselektroden in gesteuerter Weise mit einer einstellbaren Stimulationsintensität abzugeben sind, und eine Schallverarbeitungseinheit zum Erzeugen eines Stimulationsintensitätssteuersignals für jede Elektrode in Abhängigkeit von Frequenzbereichen, die jeder einzelnen Elektrode zugeordnet sind, und einer jeweiligen Signalstärke eines Eingangssignals in einem jeweiligen Frequenzbereich umfasst, wobei
die Passvorrichtung eine grafische Anzeige und eine Benutzerschnittstelle, die Eingabemittel bereitstellt, umfasst, wobei die Passvorrichtung zu Folgendem konfiguriert ist:
Daten an und/oder von dem Cochlea-Implantat zu kommunizieren, wobei die Daten für jede Stimulationselektrode des Cochlea-Implantats Parameterwerte, die einen Frequenzbereich definieren, und Parameterwerte, die mindestens zwei Stimulationsintensitätspegel definieren, einschließlich eines maximalen komfortablen Stimulationsintensitätspegels und eines minimalen Schwellenpegels (T-Pegels) für jede individuelle Stimulationselektrode des Cochlea-Implantats, darstellen, wobei der minimale Intensitätspegel die minimale Intensität bezeichnet, bei der der Benutzer ein durch die Stimulationsimpulse bewirktes Schallempfinden bemerkt, und der maximale Intensitätspegel die maximale Stimulation bezeichnet, die angewendet werden kann, ohne dem Benutzer Unbehagen zu bereiten,
auf der Anzeige für jede Elektrode eine grafische Darstellung der Parameterwerte, die einen Stimulationsintensitätsbereich und einen Frequenzbereichs in einer zweidimensionalen Matrix definieren, anzuzeigen, wobei eine Dimension der Frequenz zugeordnet ist, während die andere Dimension der Stimulationsintensität zugeordnet ist, wobei die Stimulationsintensitätsbereiche und die Frequenzbereich aller Elektroden gleichzeitig angezeigt werden,
die Eingabemittel auszulesen und die Parameterwerte als Reaktion auf Benutzereingaben zu ändern und die grafische Darstellung entsprechend zu aktualisieren, wobei die grafische Darstellung in einem Live-Modus erzeugt wird, wobei die grafische Darstellung den momentanen Stimulationsintensitätsbereich in Echtzeit und Frequenzinhalte eines dem Patienten dargelegten Schall angibt, und
weitere Parameterwerte als Reaktion auf eine der Benutzereingaben automatisch zu adaptieren, die eine Manipulation eines Parameterwertes bewirkt, was zu reibungslosen Parameteranpassungen benachbarter Parameter führt.

2. System nach Anspruch 1, wobei die grafische Darstellung eine grafische Benutzerschnittstelle ist.

3. System nach Anspruch 1 oder 2, wobei die Passvorrichtung dazu konfiguriert ist, eine grafische Benutzerschnittstelle zu erzeugen, die eine grafische Darstellung der Parameterwerte, die einen Stimulationsintensitätsbereich und einen Frequenzbereich für eine individuelle Stimulationselektrode definieren, umfasst, wobei die einer individuellen Elektrode zugeordneten Parameter durch ein Rechteck dargestellt werden, wobei eine Dimension des Rechtecks den der Elektrode zugeordneten Frequenzbereich auf einer logarithmischen Skala darstellt, während die andere Dimension den Stimulationsintensitätsbereich auf einer linearen Skala darstellt.

4. System nach mindestens einem der Ansprüche 1 bis 3, wobei die Passvorrichtung ferner dazu konfiguriert ist, in Echtzeit einen Volumeneinheitszähler anzuzeigen, der den Eingangsschallpegel darstellt, der durch den Cochlea-Implantatschallprozessor während der Passung gemessen wird.

5. System nach mindestens einem der Ansprüche 1 bis 4, wobei die Passvorrichtung ferner dazu konfiguriert ist, einen Zeitzeilenindikator mit einem entlang einer Leiste bewegbaren Index bereitzustellen und anzuzeigen, wobei der Index durch einen Benutzer bewegbar ist, wobei das Bewegen des Indexes ein Anzeigen von Darstellungen vorheriger Parameterwerte bewirkt.

6. System nach mindestens einem der Ansprüche 1 bis 5, wobei die Passvorrichtung dazu konfiguriert ist, Benutzereingaben, die dem Ziehen grafischer Komponenten der grafischen Darstellung entsprechen, zu erfassen und Parameterwerte als Reaktion auf ein erfasstes Ziehereignis zu ändern und die grafische Darstellung entsprechend zu aktualisieren.

7. Passvorrichtung zur Passung eines Cochlea-Implantats, das eine Vielzahl von Stimulationselektroden aufweist, die zum Senden von Stimulationsimpulsen konfiguriert sind, die Passvorrichtung umfassend eine Anzeigeeinheit, eine Schnittstelleneinheit zur Datenkommunikation mit einem Cochlea-Implantat, Eingabemittel zum Eingeben von Parameterwerten zur Passung der Cochlea-Implantatdaten, die auf der Anzeigevorrichtung anzuzeigen sind, wobei die Anpassungsvorrichtung zu Folgendem konfiguriert ist:
Daten an und/oder von einem Cochlea-Implantat zu kommunizieren, wobei die Daten für jede Stimulationselektrode des Cochlea-Implantats Daten, die einen Frequenzbereich definieren, und Daten, die mindestens zwei Stimulationsintensitätspegel darstellen, einschließlich eines Schwellenpegels (T-Pegels) für jede individuelle Stimulationselektrode des Cochlea-Implantats, umfassen und
auf der Anzeige für jede Elektrode einen Stimulationsintensitätsbereich und einen Frequenzbereichs in einer zweidimensionalen Matrix anzuzeigen, wobei eine Dimension der Frequenz zugeordnet ist, während die andere Dimension der Stimulationsintensität zugeordnet ist, und wobei die Parameterwerte einen minimalen Intensitätspegel und einen maximalen komfortablen Stimulationsintensitätspegel beinhalten, wobei der minimale Intensitätspegel die minimale Intensität bezeichnet, bei der der Benutzer ein durch die Stimulationsimpulse bewirktes Schallempfinden bemerkt, und der maximale Intensitätspegel die maximale Stimulation bezeichnet, die angewendet werden kann, ohne dem Benutzer Unbehagen zu bereiten, wobei die Stimulationsintensitätsbereiche und die Frequenzbereich aller Elektroden gleichzeitig angezeigt werden,
die Eingabemittel auszulesen und die Parameterwerte als Reaktion auf Benutzereingaben zu ändern und die grafische Darstellung entsprechend zu aktualisieren, wobei die grafische Darstellung in einem Live-Modus erzeugt wird, wobei die grafische Darstellung den momentanen Stimulationsintensitätsbereich in Echtzeit und Frequenzinhalte eines dem Patienten dargelegten Schall angibt, und
weitere Parameterwerte als Reaktion auf eine der Benutzereingaben automatisch zu adaptieren, die eine Manipulation eines Parameterwertes bewirkt, was zu reibungslosen Parameteranpassungen benachbarter Parameter führt.

## Revendications

1. Système pour ajuster un implant cochléaire qui possède une pluralité d'électrodes de stimulation configurées pour émettre des impulsions de stimulation, ledit système comprenant un dispositif d'ajustement et un implant cochléaire connecté en communication audit dispositif d'ajustement, ledit implant cochléaire comprenant un fil d'électrode avec une pluralité d'électrodes de stimulation formant un réseau d'électrodes, une unité de stimulation pour générer des impulsions de stimulation à émettre par l'intermédiaire des électrodes de stimulation individuelles de manière commandée avec une intensité de stimulation réglable et une unité de traitement de son destinée à générer un signal de commande d'intensité de stimulation pour chaque électrode en fonction de plages de fréquences attribuées à chaque électrode individuelle et d'une intensité de signal respective d'un signal d'entrée dans une plage de fréquences respective,
ledit dispositif d'ajustement comprenant un afficheur graphique et une interface utilisateur fournissant un moyen d'entrée, ledit dispositif d'ajustement étant configuré pour :
communiquer des données vers et/ou à partir dudit implant cochléaire, lesdites données représentant, pour chaque électrode de stimulation de l'implant cochléaire, des valeurs de paramètres définissant une plage de fréquences et des valeurs de paramètres définissant au moins deux niveaux d'intensité de stimulation, comprenant un niveau d'intensité de stimulation maximal confortable et un niveau seuil minimal (niveau T) pour chaque électrode de stimulation individuelle de l'implant cochléaire, où le niveau d'intensité minimal indique l'intensité minimale à laquelle l'utilisateur remarquera une sensation sonore entraînée par les impulsions de stimulation, et le niveau d'intensité maximal indiquant la stimulation maximale pouvant être appliquée sans gêner à l'utili sateur,
afficher sur ledit afficheur, pour chaque électrode, une représentation graphique desdites valeurs de paramètre qui définissent une plage d'intensité de stimulation et une plage de fréquences dans une matrice bidimensionnelle, une dimension étant attribuée à une fréquence tandis que l'autre dimension est attribuée à l'intensité de stimulation,
lesdites plages d'intensités de stimulation et lesdites plages de fréquences de toutes les électrodes étant affichées simultanément,
lire ledit moyen d'entrée et la modification desdites valeurs de paramètres en réponse aux entrées de l'utilisateur et mettre à jour ladite représentation graphique en conséquence, et la représentation graphique est générée en mode en direct, ladite représentation graphique indiquant momentanément la plage d'intensités de stimulation en temps réel et les contenus en fréquences d'un son qui est présenté au patient, et pour adapter automatiquement des valeurs de paramètre supplémentaires en réponse à l'une des entrées d'utilisateur qui entraîne une manipulation d'une valeur de paramètre, aboutissant à des réglages de paramètres lisses des paramètres voisins.

2. Système selon la revendication 1, ladite représentation graphique étant une interface utilisateur graphique.

3. Système selon la revendication 1 ou 2, ledit dispositif d'ajustement étant configuré pour générer une interface utilisateur graphique comprenant une représentation graphique desdites valeurs de paramètres qui définissent une plage d'intensités de stimulation et une plage de fréquences pour une électrode de stimulation individuelle, lesdits paramètres attribués à une électrode individuelle étant représentés par un rectangle, une dimension dudit rectangle représentant la plage de fréquences attribuée à ladite électrode sur une échelle logarithmique alors que l'autre dimension représente la plage d'intensités de stimulation sur une échelle linéaire.

4. Système selon au moins l'une des revendications 1 à 3, ledit dispositif d'ajustement étant en outre configuré pour afficher en temps réel un compteur d'unités de volume représentant le niveau sonore d'entrée mesuré par le processeur de son d'implant cochléaire durant l'ajustement.

5. Système selon au moins l'une des revendications 1 à 4, ledit dispositif d'ajustement étant en outre configuré pour fournir et afficher un indicateur de ligne de temps avec un index mobile le long d'une barre, ledit index étant déplaçable par un utilisateur, ledit déplacement dudit index entraînant l'affichage de représentations de valeurs de paramètre précédentes.

6. Système selon au moins l'une des revendications 1 à 5, ledit dispositif d'ajustement étant configuré pour détecter une entrée utilisateur qui correspond au glissement de composants graphiques de ladite représentation graphique et pour modifier des valeurs de paramètre en réponse à un événement de glissement détecté et pour mettre à jour ladite représentation graphique en conséquence.

7. Dispositif d'ajustement destiné à ajuster un implant cochléaire qui possède une pluralité d'électrodes de stimulation configurées pour émettre des impulsions de stimulation, ledit dispositif d'ajustement comprenant une unité d'affichage, une unité d'interface pour la communication de données avec un implant cochléaire, un moyen d'entrée pour entrer des valeurs de paramètre en vue de l'ajustement des données implant cochléaire à afficher sur ledit dispositif d'affichage, ledit dispositif d'ajustement étant configuré
pour communiquer des données vers et/ou à partir d'un implant cochléaire, lesdites données comprenant, pour chaque électrode de stimulation de l'implant cochléaire, des données définissant une plage de fréquences et des données représentant au moins deux niveaux d'intensité de stimulation, comprenant un niveau seuil (niveau T) pour chaque électrode de stimulation individuelle de l'implant cochléaire et
pour afficher sur ledit afficheur, pour chaque électrode, une plage d'intensités de stimulation et une plage de fréquences dans une matrice bidimensionnelle, une dimension étant attribuée à la fréquence alors que l'autre dimension est attribuée à l'intensité de stimulation, et où lesdites valeurs de paramètre comprennent un niveau d'intensité minimal et un niveau d'intensité de stimulation maximal confortable, où le niveau d'intensité minimal indique l'intensité minimale à laquelle l'utilisateur remarquera une sensation sonore entraînée par les impulsions de stimulation, et le niveau d'intensité maximal indique la stimulation maximale qui peut être appliquée sans entraîner d'inconfort à l'utilisateur, lesdites plages d'intensités de stimulation et lesdites plages de fréquences de toutes les électrodes étant affichées simultanément,
pour lire ledit moyen d'entrée et la modification desdites valeurs de paramètre en réponse aux entrées d'utilisateur et mettre à jour ladite représentation graphique en conséquence, et la représentation graphique est générée en mode en direct, ladite représentation graphique indiquant momentanément la plage d'intensités de stimulation en temps réel et les contenus en fréquences d'un son présenté au patient, et
pour adapter automatiquement des valeurs de paramètre supplémentaires en réponse à l'une des entrées de l'utilisateur qui entraîne une manipulation d'une valeur de paramètre, aboutissant à des ajustements de paramètres lisses des paramètres voisins.
